# EUROPEAN PATENT APPLICATION

(11) **EP 2 168 988 A1**
(43) Date of publication of application: **31.03.2010**
(21) Application number: 08790876.0
(22) Date of filing: 04.07.2008
(51) Int. Cl.: C08F 10/00, A61K 8/26, A61K 8/29, A61K 8/81, B01D 39/16, C08F 4/654, C08J 9/24, C08L 21/00, C08L 23/02

(54) **SUPER HIGH MOLECULAR WEIGHT POLYOLEFIN FINE PARTICLE, METHOD FOR PRODUCING THE SAME AND MOLDED BODY OF THE SAME**

(30) Priority: 13.07.2007 JP 2007184362
(71) Applicant: Mitsui Chemicals, Inc., Tokyo 105-7117 (JP)
(72) Inventor: YASUDA, Kazuaki, Kuga-gun Yamaguchi 740-0061 (JP); MATSUMOTO, Tetsuhiro, Iwakuni-shi Yamaguchi 740-1213 (JP); MIZUMOTO, Kunihiko, Ichihara-shi Chiba 299-0108 (JP)
(74) Representative: HOFFMANN EITLE
(86) International application number: PCT/JP2008/062145
(87) International publication number: WO 2009/011231

(57) **Abstract**

[Subject]

The present invention provides ultra-high molecular weight polyolefin fine particles having a small particle diameter and a narrow particle size distribution, a molded article made of the fine particles and a sintered filter obtainable by sintering molding the ultra-high molecular weight polyolefin fine particles.

[Means for solving the subject]

The ultra-high molecular weight polyolefin fine particles comprising 1 to 50 ppm of a titanium atom and 1 to 1000 ppm of an aluminum atom, wherein the ultra-high molecular weight polyolefin fine particles have:
[A] an intrinsic viscosity, as measured in decalin at 135°C, of not less than 5 dl/g,
[B] an average particle diameter of not more than 20 µm, and
[C] a content of particles having a particle diameter of 0 to 40 µm of not less than 90 % by mass.

## Description

### FIELD OF THE INVENTION

The present invention relates to ultra-high molecular weight polyolefin fine particles having a narrow particle size distribution and a small average particle diameter.

### TECHNICAL BACKGROUND

Ultra-high molecular weight polyolefins represented by ultra-high molecular weight polyethylene are light in weight and have excellent abrasion resistance, impact resistance, chemical resistance and self-lubricating properties. Therefore, they have been used for various uses such as mechanical parts, lining materials, sporting goods and the like.

In the meantime, when a filter is molded from ultra-high molecular weight polyolefins, the molding is conducted by a rotation molding method or a powder molding method such that polyolefin powder is molded as it is, instead of a usual melt molding because the ultra-high molecular weight polyolefins have inferior fluidity in melting. The resulting filter, however, does not have sufficient filtering characteristics.

As is clear from observation of similar molded articles made of metals, it is considered that resins used for powder molding preferably have a smaller powdery particle diameter and a narrower particle size distribution in view of uniformities and physical properties of molded articles. However, since polyolefin powder is light in weight and further is easily charged with static electricity, agglomeration of powder is caused. Accordingly, it has been difficult to produce ultra-high polyolefin fine particles having an average particle diameter of not more than 20 µm and having a narrow particle size distribution efficiently.

Until now, there halve been polyolefin powders (compositions) having a specific particle size distribution (referred to Patent Documents 1 and 2). But, the small size of the average particle diameter and the narrow width of the particle size distribution in the powders are not sufficient.
Moreover, a process for preparing a fine particle polyethylene having an ultra-high molecular weight using a specific catalyst system is known (referred to Patent Document 3). However, the process is not sufficient in performance because a large amount of catalyst residues is caused.

Meanwhile, in a slurry (suspension) polymerization method, it has been an important subject such that when polyethylene is produced, generation of fouling in an inner wall of a polymerization vessel is prevented (referred to Patent Document 4).
Patent Document 1: JP-A-S60(1985)-163935
Patent Document 2: JP-A-S62(1987)-1736
Patent Document 3: JP-A-2006-206769
Patent Document 4: JP-B-H7(1995)-64893

### DISCLOSURE OF INVENTION

### SUBJECT TO BE SOLVED BY THE INVENTION

It is an object of the present invention to provide ultra-high molecular weight polyolefin fine particles having a small particle diameter and a narrow particle size distribution, it is another object of the invention to provide a molded article formed from the ultra-high molecular weight polyolefin fine particles, and it is a further object of the invention to provide a sintered filter obtainable by sintering molding the ultra-high molecular weight polyolefin fine particles.

### MEANS FOR SOLVING THE SUBJECT

The present inventors have been earnestly studied on ultra-high molecular weight polyolefin fine particles in view of the above circumstances, and found a process for producing efficiently ultra-high molecular weight polyolefin fine particles having a narrow particle size distribution and a small average particle diameter. Thus, the present invention has been accomplished.

That is to say, the present invention contains the following subjects.
[1] The ultra-high molecular weight polyolefin fine particles of the present invention comprise 1 to 50 ppm of a titanium atom and 1 to 1000 ppm of an aluminum atom, wherein the ultra-high molecular weight polyolefin fine particles have:
   [A] an intrinsic viscosity [η], as measured in decalin at 135°C, of not less than 5 dl/g,
   [B] an average particle diameter of not more than 20 µm, and
   [C] a content of particles having a particle diameter of 0 to 40 µm of not less than 90 % by mass.
[2] The ultra-high molecular weight polyolefin fine particles according to [1] are obtainable by using a catalyst comprising:
   (A) a solid transition metal catalyst component comprising a titanium atom and a magnesium atom, and
   (B) an organoaluminum compound.
[3] The ultra-high molecular weight polyolefin fine particles according to [1] are obtainable by regulating the amount of a resulting polymer to not more than 7000 g per 1 g of the solid transition metal catalyst component (A) by using a catalyst comprising:
   (A) a solid transition metal catalyst component comprising a titanium atom and a magnesium atom, and
   (B) an organoaluminum compound.
[4] The ultra-high molecular weight polyolefin fine particles according to any one of [1] to [3] are obtainable by using a polymerization reactor equipped with a stirring blade and regulating a peripheral speed of a stirring blade tip to not less than 3.0 m/sec.
[5] The ultra-high molecular weight polyolefin fine particles according to any one of [1] to [4], are **characterized in that** the polyolefin is an ethylene polymer.
[6] A process for producing the ultra-high molecular weight polyolefin fine particles as defined in [2] wherein the amount of a resulting polymer is regulated to be not more than 7000 g per 1 g of the solid transition metal catalyst component (A).
[7] The process for producing the ultra-high molecular weight polyolefin fine particles according to [6] is **characterized in that** a polymerization reactor equipped with a stirring blade is used and the peripheral speed of a stirring blade tip is regulated to not less than 3.0 m/sec.
[8] The process for producing ultra-high molecular weight polyolefin fine particles according to [6] or [7] is **characterized in that** the polyolefin is an ethylene polymer.
[9] The molded article of the present invention comprises the ultra-high molecular weight polyolefin fine particles according to any one of [1] to [5].
[10] The sintered filter of the present invention is obtainable by sintering molding the ultra-high molecular weight polyolefin fine particles according to any one of [1] to [5].
[11] The modifier for rubber of the present invention comprises the ultra-high molecular weight polyolefin fine particles according to any one of [1] to [5].
[12] The binder for active carbon of the present invention comprises the ultra-high molecular weight polyolefin fine particles according to any one of [1] to [5].
[13] The cosmetic composition of the present invention comprises the ultra-high molecular weight polyolefin fine particles according to any one of [1] to [5].

### EFFECT OF THE INVENTION

The ultra-high molecular weight polyolefin fine particles of the present invention can be produced efficiently because they have a small amount of catalyst residues, and agglomeration of powder and fouling to the inner wall of a polymerization vessel are not caused in the production thereof.

When a molded article is blended or the surface thereof is covered with the ultra-high molecular weight polyolefin fine particles according to the present invention, the molded article can be improved in sliding properties and abrasion properties. Furthermore, in the use of sintered filters and the like, the ultra-high molecular weight polyolefin fine particles according to the present invention can provide molded articles having no uneven molding, homogeneity and excellent filtering characteristics because of having excellent processability.

### BEST MODE FOR CARRYING OUT THE INVENTION

### [Ultra-high molecular weight polyolefin fine particles]

The ultra-high molecular weight polyolefin fine particles according to the present invention may be a homopolymer such as polyethylene, polypropylene, poly-1-butene, poly-4-methyl-1-pentene, and further a copolymer of ethylene and a small amount of other α-olefins such as propylene, 1-butene, 1-hexene, 1-octene and 4-methyl-1-pentene, preferably an ethylene polymer, particularly preferably polyethylene.

The ultra-high molecular weight polyolefin fine particles according to the present invention have an intrinsic viscosity [n], as measured in decalin at 135°C, of not less than 5 dl/g, preferably 5 to 50 dl/g, more preferably 5 to 30 dl/g. The intrinsic viscosity is preferably in the above range, because they have excellent abrasion resistance, impact resistance, chemical resistance and self-lubricating properties.

The ultra-high molecular weight polyolefin fine particles according to the present invention comprise, as a catalyst residue (Ash) component, a titanium atom in an amount of 1 to 50 ppm, preferably 1 to 30 ppm, and an aluminum atom in an amount of 1 to 1000 ppm, preferably 1 to 300 ppm.

The ultra-high molecular weight polyolefin fine particles according to the present invention have an average particle diameter of not more than 20 µm, preferably not more than 15 µm, more preferably 5 to 15 µm. The average particle diameter is preferably not more than 20 µm, because the filter pore diameter can be decreased. On the other hand, the average particle diameter is preferably not less than 5 µm because particles can be easily handled in the molding.

In the particle size distribution of the ultra-high molecular weight polyolefin fine particles according to the present invention, particles having a particle diameter of from 0 to 40 µm (namely, not more than 40 µm) are present in an amount of not less than 90 % by mass, preferably 92 to 98 % by mass, and particles having a particle diameter of from 1 to 40 µm are present in an amount of not less than 90 % by mass, preferably 92 to 98 % by mass. The particles having a particle diameter of 0 to 40 µm are preferably present in an amount of not less than 90 % by mass, because the particle size distribution is narrow and a filter having uniform pore diameters can be prepared efficiently.

### [Production process for ultra-high molecular weight polyolefin fine particles]

The ultra-high molecular weight polyolefin fine particles according to the present invention can be produced in the following process.
That is to say, the ultra-high molecular weight polyolefin fine particles can be produced by polymerizing an olefin using a catalyst which comprises a solid transition metal catalyst component (A) essentially containing a compound of a titanium atom and a magnesium atom, and an organoaluminum compound (B).

Moreover, a small amount of an α-olefin may be pre-polymerized using the solid transition metal catalyst component (A) in an inert hydrocarbon medium such as saturated aliphatic hydrocarbon and the like, to form a catalyst which is more finely divided than the solid transition metal catalyst component (A), and then olefin polymerization may be carried out.

The solid transition metal catalyst component (A) used in the present invention essentially comprises magnesium, a transition metal (for example, titanium, vanadium, chromium, zirconium, etc.) and a halogen. The component (A) has an atomic ratio of magnesium to transition metal (magnesium/transition metal) of usually from 2 to 100, particularly preferably 4 to 70, and an atomic ratio of halogen to transition metal (halogen/transition metal) of usually from 4 to 100, particularly preferably 6 to 40.

Such a solid transition metal catalyst component (A) may contain other elements, metals, functional groups and electron donors except for the above essential components.
The solid transition metal compound catalyst component (A) has a specific surface area of usually not less than 3 m²/g, preferably not less than 40 m²/g, more preferably 100 to 800 m²/g.

With regard to the production process for the solid transition metal compound catalyst component (A), a lot of processes have been proposed already. Basically, there are a process of directly allowing a magnesium compound to react with a transition metal compound, a process of reacting in the presence of an electron donor or other reactive agents, for example, in the presence of a compound of silicon and aluminum or the like, and a process of previously allowing any one or both of a magnesium compound and a transition metal compound to contact with an electron donor or other reactive agents and then reacting both of them. Typical examples of the processes are disclosed in, for example, JP-B-S47(1972)-41676, JP-B-S47(1972)-46269, JP-A-S49(1974)-72383, JP-B-S50(1975)-32270, JP-A-S50(1975)-108385, JP-A-S50(1975)-126590, JP-A-S51(1976)-20297, JP-A-S51(1976)-28189, JP-A-S51(1976)-64586, JP-A-S51(1976)-92885, JP-A-S51(1976)-136625, JP-A-S52(1977)-87489, JP-A-S52(1977)-100596, JP-A-S52(1977)-147688, JP-A-S52(1977)-104593, JP-A-S53(1978)-43094, JP-B-S53(1978)-46799, JP-A-S55(1980)-135102, JP-A-S55(1980)-135103, JP-A-S56(1981)-811 and JP-A-S56(1981)-11908.

Of these processes, some examples will be simply described below.
(1) A magnesium compound having a large specific surface area is reacted with a transition metal compound.
(2) A magnesium compound is allowed to contact with a transition metal compound by co-grinding in the presence or absence of an electron donor, a grinding assistant or the like.
(3) A magnesium compound is previously allowed to contact with an electron donor, and thereafter, to contact with a reaction assistant such as an organoaluminum compound or a halogen-containing silicon compound or not to contact, and then reacting with a titanium compound.
(4) The resulting compound prepared in any one of the processes (1) to (3) is further allowed to react with at least one selected from an electron donor, an organoaluminum compound and a titanium compound.
(5) The resulting compound prepared in any one of the processes (1) to (4) is washed sufficiently with a hydrocarbon and a halogenated hydrocarbon. In the process, it is preferred to use the solid transition metal compound catalyst component (A) prepared by the process (3). That is to say, the solid transition metal compound catalyst component (A) obtainable from the titanium compound, the magnesium compound and the electron donor is preferred.

Examples of the magnesium compound used in preparation of the solid transition metal catalyst component (A) are magnesium oxide, magnesium hydroxide, hydrotalcite, carboxylic acid magnesium salt, alkoxymagnesium, allyloxymagnesium, alkoxymagnesium halide, allyloxymagnesium halide, magnesium dihalides such as anhydrous magnesium chloride, organic magnesium compounds and compounds obtainable by treating the organic magnesium compound with an electron donor, halosilane, alkoxysilane, silanol or an aluminum compound.

Examples of the electron donor used in preparation of the solid transition metal catalyst component (A) are alcohol, phenols, ketone, aldehyde, esters of organic acid or inorganic acid such as carboxylic acid and the like, oxygen-containing electron donors such as ether, acid amide, acid anhydride, alkoxysilane and the like, and nitrogen-containing electron donors such as ammonia, amine, nitrile, isocyanate and the like. Specific examples are alcohols having 1 to 18 carbon atoms such as methanol, ethanol, propanol, pentanol, hexanol, octanol, 2-ethylhexanol, dodecanol, octadecyl alcohol, benzyl alcohol, phenylethyl alcohol, cumyl alcohol, isopropylbenzyl alcohol, etc;
phenols having 6 to 25 carbon atoms and optionally having an alkyl group such as phenol, cresol, xylenol, ethylphenol, propylphenol, cumylphenol, nonylphenol, naphthol, etc;
ketones having 3 to 15 carbon atoms such as acetone, methyl ethyl ketone, methyl isobutyl ketone, acetophenone, benzophenone, etc;
aldehydes having 2 to 15 carbon atoms such as acetaldehyde, propionaldehyde, octyl aldehyde, benzaldehyde, tolualdehyde, naphthaldehyde, etc;
organic acid esters having 2 to 30 carbon atoms such as methyl formate, methyl acetate, ethyl acetate, vinyl acetate, propyl acetate, octyl acetate, cyclohexyl acetate, ethyl propionate, methyl butyrate, ethyl valerate, ethyl stearate, methyl chloroacetate, ethyl dichloroacetate, methyl methacrylate, ethyl crotonate, dibutyl maleate, diethyl butylmalonate, diethyl dibutylmalonate, ethyl cyclohexanecarboxylate, diethyl 1,2-cyclohexanedicarboxylate, di-2-ethylhexyl 1,2-cyclohexanedicarboxylate, ethyl benzoate, propyl benzoate, butyl benzoate, octyl benzoate, cyclohexyl benzoate, phenyl benzoate, benzyl benzoate, methyl toluylate, ethyl toluylate, amyl toluylate, ethyl ethylbenzoate, methyl anisate, ethyl anisate, ethyl ethoxybenzoate, dimethyl phthalate, diethyl phthalate, dibutyl phthalate, dioctyl phthalate, monobutyl phthalate, dibutyl naditate, γ-butyrolactone, δ-valerolactone, coumarin, phthalide, ethylene carbonate, etc;
alkoxysilanes such as ethyl silicate, butyl silicate, vinyltriethoxysilane, etc;
acid halides having 2 to 15 carbon atoms such as acetyl chloride, benzoyl chloride, toluoyl chloride, anisoyl chloride, phthaloyl dichloride, etc;
ethers having 2 to 20 carbon atoms such as methyl ether, ethyl ether, isopropyl ether, butyl ether, aminoether, tetrahydrofuran, anisole, diphenyl ether, etc;
acid amides such as acetamide, benzamide, toluamide, etc;
acid anhydrides such as benzoic anhydride, phthalic anhydride, etc;
amines such as methylamine, ethylamine, diethylamine, tributylamine, piperidine, tribenzylamine, aniline, pyridine, picoline, tetramethylethylenediamine, 2,2,6,6-tetramethylpiperidine, etc; and
nitriles such as acetonitrile, benzonitrlle, tolunitrile, etc. These electron donors may be used singly, or two or more may be used in combination.

Examples of the transition metal compound used in preparation of the solid transition metal catalyst component (A) may include titanium, vanadium, chromium, zirconium, hafnium and the like. These may be used singly or two or more may be used in combination. Of the above transition metal compounds, the titanium compound or the vanadium compound is preferable, and particularly the titanium compound is preferably used. Specific examples thereof are titanium compounds represented by Ti(OR)ₙX₄₋ₙ wherein R is a hydrocarbon group, X is halogen and 0≤n≤4, such as TiCl₄, TiBr₄, TiI₄, Ti(OCH₃)Cl₃, Ti(OC₂X₅)Cl₃, Ti(OC₆H₅)Cl₃, Ti(OC₂H₅)2Cl₂, Ti(OC₃H₇)₂Cl₂, Ti(OC₂H₅)₃Cl, Ti(OC₆H₅)₃Cl, Ti(OC₂H₅)₄, Ti(OC₃H₇)₄, Ti(OC₄H₉)₄, Ti(OC₆H₁₃)₄, Ti(OC₆H₁₁)₄, Ti(OC₈H₁₇)₄, Ti[OCH₂(C₂H₅)CHC₄H₉]₄, Ti(OC₉H₁₉)₄, Ti[O₆H₃(CH₃)₂]₄, Ti(OCH₃)₂(OC₄H₉)₂, Ti(OC₃H₇)₃(OC₄H₉), Ti(OC₂H₅)₂(OC₄H₉)₂, Ti(OC₂H₄Cl)₄, Ti(OC₂H₄OCH₃)₄, etc. As far as the titanium compound has a low valence, it may be in any one of crystal systems. Specific examples thereof are TiCl₃•T type compounds obtainable by reducing titanium tetrachloride with a titanium metal, TiCl₃•A type compounds obtainable by reducing with an aluminum metal, TiCl₃•H type compounds obtainable by reducing with hydrogen, titanium trihalide such as TiCl₃ obtainable by reducing with an organoaluminum compound represented by (C₂H₅)₃Al, (C₂H₅)₂AlCl or (C₂H₅)_{1.5}AlCl_{1.5}, alkoxytitanium (III) compounds such as Ti(OCH₃)₃, Ti(OC₂H₅)₃, Ti(OnC₄H₉)₃, Ti(OCH₃)Cl₂•2CH₃OH or Ti(OCH₃)₂Cl•CH₃OH, and TiCl₂ obtainable by reducing TiCl₃ with hydrogen. Moreover, the transition metal compound, which is in a solid state at ordinary temperature such as titanium trichloride or titanium dichloride may be subjected to liquefying treatment and then may be used.

As the above vanadium compound, a compound represented by VO(OR)ₘX₃₋ₘ (R and X are the same as defined above, and 0≤m≤3) or VXₚ (2≤p≤4) is generally used, and specific examples thereof may include VOCl₃, VO(OC₂H₅)Cl₂, VO(OC₂H₅)₃, VO((DC₂H₅) VO(OC₄H₉)₃, vO[OCH₂(CH₂)CHC₄H₉]₃, VCl₄, VCl₃, VCl₂, etc.

As the organoaluminum compound used in preparation of the solid transition metal catalyst component (A), the organoaluminum compound (B) as described later can be used.
Examples of the halogen-containing silicon compound used in preparation of the solid transition metal component (A) may include tetrahalosilane, alkoxyhalosilane, alkylhalosilane and halogenated polysiloxane.

The organoaluminum compound (B) used in the present invention may be a compound at least having one Al-carbon bond in one molecule, and examples thereof may include an organoaluminum compound represented by the formula (i) R¹ₘAl(OR²)ₙHₚX_{q}wherein R¹ and R² are each a hydrocarbon group having usually 1 to 15 carbon atoms, preferably 1 to 4 carbon atoms such as alkyl group, alkenyl group, aryl group, etc, and they may be the same or different, X is a halogen, and m, n, p and q satisfy the followings, 0<m≤3, 0≤n<3, 0≤p<3, 0≤q<3, and m + n + p + q = 3, and an organoaluminum compound represented by the formula (ii) M¹AlR¹₄ wherein M¹ is Li, Na or K, and R¹ is the same as defined in (i), namely, an alkylated complex compound of a Group 1 metal and aluminum.

Examples of the organoaluminum compound represented by the formula (i) may include a compound represented by R¹ₘAl(OR²)₃₋ₘ wherein R¹ and R² are the same as above and m preferably satisfies 1.5≤m<3, a compound represented by R¹ₘAlX₃₋ₘ wherein R¹ is the same as above, X is a halogen and m preferably satisfies 0<m<3, a compound represented by R¹ₘAlH₃₋ₘ wherein R¹ is the same as above and m preferably satisfies 2≤m<3, and a compound represented by R¹ₘAl(OR²)ₙX_{q} wherein R¹ and R² are the same as above, X is a halogen and 0<m≤3, 0≤n<3, 0≤q<5 and m + n + q = 3.
Specific examples of the aluminum compound represented by the formula (i) are:
trialkylaluminums such as triethylaluminum, tributylaluminum, triisobutylaluminum and trihexylaluminum;
trialkenylaluminums such as triisoprenylaluminum etc;
dialkylaluminum alkoxides such as diethylaluminum ethoxide and dibutylaluminum butoxide;
alkylaluminum sesquialkoxides such as ethylaluminum sesquiethoxide and butylaluminum sesquibutoxide;
partially alkoxylated alkylaluminum having an average composition represented by R¹_{2.5}Al(OR²)_{0.5};
dialkylaluminum halides such as diethylaluminum chloride, dibutylaluminum chloride and diethylaluminum bromide;
alkylaluminum sesquibromides such as ethylaluminum sesquichloride, butylaluminum sesquichloride and ethylaluminum sesquibromide;
partially halogenated alkylaluminum such as alkylaluminum dihalides e.g. ethylaluminum dichloride, propylaluminum dichloride or butylaluminum dibromide;
partially hydrogenated alkylaluminums such as dialkylaluminum hydrides e.g. diethylaluminum hydride and dibutylaluminum hydride, ethylaluminum dihydride, and propylaluminum dihydride; and
partially alkoxylated or halogenated alkyl aluminums such as ethylaluminum ethoxychloride, butylaluminum butoxychloride and ethylaluminum ethoxybromide.
Examples of compounds similar to the compounds represented by the formula (i) may include organoaluminum compounds in which at least two aluminums are bonded through an oxygen atom or a nitrogen atom. These compounds may include compounds represented by the following chemical formulas (1) to (3).

(C₂H₅)₂AlOAl(C₂H₅)₂ (1)

(C₄H₉)₂AlOAl(C₄H₉)₂ (2)

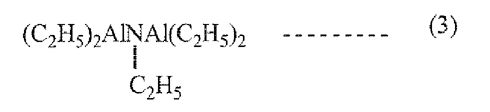

Examples of the organcaluminum compound represented by the formula (ii) are LiAl(C₂H₅)₄ and LiAl(C₇H₁₅)₄. Among them, it is particularly preferred to use trialkylaluminum, alkylaluminum halide and mixtures thereof.

In order to prepare the ultra-high molecular weight polyolefin fine particles having an average particle diameter of not more than 20 µm and containing 90 or more % by mass of particles having a particle diameter of 0 to 40 µm according to the present invention using the catalyst which comprises the solid transition metal catalyst component (A) essentially containing the magnesium compound and the transition metal compound, and the organoaluminum compound (B), the solid transition metal catalyst component (A) is preferably regulated to have an average particle diameter of usually 0.1 to 10 µm, particularly 0.1 to 5 µm and then is submitted to use. For example, the particle diameter of the solid transition metal catalyst component (A) can be regulated in accordance with the preparation process of a solid titanium catalyst component as disclosed in JP-A-S56 (1981)-811 which process comprises allowing a liquid magnesium compound to contact with a liquid titanium compound and regulating the depositing condition in depositing a solid product.

For example, in the above preparation process of the solid titanium catalyst component, a hydrocarbon solution in which magnesium chloride and higher alcohol are dissolved, and titanium tetrachloride are mixed at a low temperature and thereafter the temperature is elevated to about 50 to 100°C, and the solid product is deposited with vigorous stirring. In this deposition, the particle diameter of the solid catalyst component can be regulated by controlling the stirring condition.

When the polymerization procedure carried out in the above specific conditions using the specific solid transition metal catalyst (A), the ultra-high molecular weight polyolefin fine particles having a small particle diameter and a narrow particle size distribution can be prepared efficiently.

In the present invention, using the particulate solid transition metal catalyst component (A), olefin homo-polymerization, copolymerization of olefins or copolymerization of an olefin with a polymerizable monomer (for example polyene) can be carried out. As a result, not only high crystalline polymers but also low crystalline polymers and non-crystalline polymers can be prepared. Examples of the olefins used in the present invention are ethylene, propylene, 1-butene, 1-pentene, 1-hexene, 1-octene, 1-decene, 1-dodecene, 1-tetradecene, 1-octadecene, 3-methyl-1-pentene, 4-znethyl-1-pentene, 4,4-dimethyl-1-pentene, 3,3-dimethyl-1-butene and styrene. Particularly, ethylene is preferable. Moreover, of the polymerizable monomers, examples of the polyene are butadiene, isoprene, 1,4-hexadiene, 1,7-octadiene, 1,3,7-octatriene, 2,4,6-octatriene, 5-ethylidene-2-norbornene, 5-vinyl-2-norbornene and dicyclopentadiene.

The polymerization may be carried out by any of continuous polymerization and batch polymerization. As the polymerization method, a liquid phase polymerization method such as dissolution polymerization and slurry polymerization (suspension polymerization), and a gas phase polymerization method can be used, particularly the slurry polymerization method is preferable.
Using the slurry polymerization method, the ultra-high molecular weight polyolefin fine particles of the present invention can be stably prepared with a good catalyst efficiency. In carrying out the slurry polymerization, the solid transition metal catalyst component (A) is used in an amount in terms of transition metal of usually 0.0001 to 0.1 mm mol, preferably 0.001 to 0.1 mm mol per 1 L of the polymerization medium. Moreover, the organoaluminum compound (B) can be used additionally during the polymerization reaction. In the addition, the organoaluminum compound (B) is preferably used in an amount such that the atomic ratio of Al to transition metal in the polymerization system is usually 1 to 1000, particularly 1 to 500.

The polymerization temperature, which differs according to the kind of olefin or the polymerization method, is usually 0 to 300°C, preferably 0 to 200°C. The polymerization reaction can be carried out at atmospheric pressure or under a pressurized condition, for example, at a pressure of 1 to 3000 kg/cm², preferably 1 to 2000 kg/cm².

In carrying out the slurry polymerization, an inert hydrocarbon may be used or the monomer olefin itself may be used as the polymerization medium. Particularly, the polymerization medium is preferably a saturated aliphatic hydrocarbon or the olefin itself which is the same as the monomer. Furthermore, a molecular weight modifier such as hydrogen may be used with the aim of regulating the molecular weight. Moreover, a halogenated hydrocarbon, a metal halide or an electron donor may be used with the aim of improving the catalyst activity, regulating the molecular weight distribution or controlling the stereo-regularity. The above-described compounds can be appropriately used as the electron donor.

Since the ultra-high molecular weight polyolefin fine particles having an average particle diameter of not more than 20 µm and a narrow particle size distribution are fine powder, fouling occasionally generates by aggregation. In this case, the fouling generation can be decreased by increasing the stirring ability of a polymerization reactor. The peripheral speed of the stirring blade tip, which depends on the polymerization method, is not less than 3.0 m/sec, preferably not less than 3.5 m/sec, because adhesion of powder to the polymerization reactor wall surface can be depressed effectively.

In order to prepare the ultra-high molecular weight polyolefin fine particles having an average particle diameter of not more than 20 µm and containing not less than 90 % by mass of particles having a particle size of 0 to 40 µm according to the present invention, it is necessary to decrease the amount of the resulting polymer to the solid transition metal catalyst component (A). The upper limit of the amount of the resulting polymer per 1g of the solid transition metal catalyst component (A) is 20000 g, preferably 10000 g, more preferably 7000 g, and the lower limit is 0 g, preferably 100 g, more preferably 500 g. More specifically, the amount of the resulting polymer per 1 g of the solid transition metal catalyst component (A) is 100 to 20000 g, preferably 100 to 10000 g, more preferably 100 to 7000 g, furthermore preferably 500 to 10000 g, particularly 500 to 7000 g. When the upper limit of the amount of the resulting polymer is over 20000 g, the average particle diameter of the resulting polyolefin fine particles is increased and the particle size distribution is occasionally widened. The amount of the resulting polymer per 1 g of the solid transition metal catalyst component (A) can be regulated by controlling the polymerization temperature, the polymerization pressure or the polymerization time.

After the polymerization, the ultra-high molecular weight polyolefin fine particles of the present invention can be subjected to deashing treatment. For example, the dashing treatment is carried out in such a way that 1 part by mass of the ultra-high molecular weight polyolefin fine particles are allowed to contact with a mixed solution which comprises 0.01 to 10 part by mass of an alcohol having 2 to 10 carbon atoms and optionally 0.01 to 10 part by mass of a chelate compound with stirring in a reaction vessel at a temperature of 20 to 150°C for a period of time of 0.01 to 10 hr, followed by filtration, cleaning and dying.

The ultra-high molecular weight polyolefin fine particles of the present invention contains, as a catalyst residue (Ash) component, a titanium atom in an amount of 1 to 50 ppm, preferably 1 to 30 ppm, and an aluminum atom in an amount of 1 to 1000 ppm, preferably 1 to 300 ppm.

The ultra-high molecular weight polyolefin fine particles of the present invention has an intrinsic viscosity [η] at 135° C in decalin of not less than 5 dl/g, preferably 5 to 50 dl/g, more preferably 5 to 30 dl/g. The intrinsic viscosity can be regulated by controlling the polymerization temperature and the amount of hydrogen fed to the polymerization reaction system. For example, the intrinsic viscosity of the resulting polymer can be increased by decreasing the polymerization temperature or decreasing the amount of hydrogen fed.

### [Molded article]

The ultra-high molecular weight polyolefin fine particles of the present invention can be formed into molded articles having various forms by various molding methods such as injection molding, extrusion molding, compression molding, powder molding or sintering molding. Moreover, various additives, which are usually added to polyolefins, may be added.

### [Sintered filter]

The sintered filter of the present invention can be prepared by filling a mold with the ultra-high molecular weight polyolefin fine particles of the present invention and heating for a prescribed time. The heating temperature is 140 to 280 ° C, preferably 50 to 250° C, and the heating time is 1 min to 4 hr, preferably 2 min to 2 hr.

Since the ultra-high molecular weight polyolefin fine particles of the present invention have a small particle diameter and a narrow particle size distribution, a sintered filter having a uniform small hole diameter and high performance can be prepared. Therefore, the sintered filter of the present invention can be suitably used for not only filters for filtering industrial water but also filters for drinking water, juices, wines, alcoholic drinks and the like.

### [Others]

In addition to the above uses, the ultra-high molecular weight polyolefin fine particles of the present invention can be suitably used for rubber modifying agents, active carbon binders, cosmetic compositions and the like.

### [Example]

Next, the present invention will be described in more detail with reference to the examples, but the present invention should not be limited by these examples.

In the present invention, the average particle diameter, the particle size distribution and the contents of a titanium atom and an aluminum atom on the ultra-high molecular weight polyolefin fine particles were determined in the following methods.

### (1) Average particle diameter (SEM measurement)

A carbon paste was applied on a sample base for SEM observation, polyolefin fine particles were put on the carbon paste and then dried. Thereafter, Pt vapor deposition was carried out using a vapor deposition device (E-1030 manufactured by Hitachi Ltd.) and a photograph at a magnification of 2000 times was taken using SEM (JEM-6300F manufactured by JEOL Ltd.). Subsequently, the longitudinal diameters of 64 particles in the photograph were measured using a vernier caliper and the arithmetic mean value thereof was taken as an average particle diameter.

### (2) Particle size distribution

Using a particle size distribution measuring device (coulter multisizer II manufactured by Beckman), a sample which was dispersed in a decane solution, namely, polyolefin fine particles was injected from a nozzle having a diameter of 200 µm, the particle diameter was determined from the sedimentation rate of the particles and was taken as a mass distribution.

### (3) The contents of titanium atom and aluminum atom in Polymer

Using a fluorescent X-ray analysis device (XRF), the contents of a titanium atom and an aluminum atom in polyolefin fine particles were measured.

### Example 1

### [Preparation of Solid transition metal catalyst component (A)]

4.76 g of anhydrous magnesium chloride, 23.2 ml of 2-ethylhexyl alcohol and 25 ml of decane were heated at 120°C for 2 hr to prepare a uniform solution. Furthermore, 0.9 ml or ethyl benzoate was added to the solution. The uniform solution was cooled to -20° C and was dropped in 200 ml of titanium tetrachloride with stirring over 1 hr. After completion of the dropping, this mixture was heated to 90° Cover 1.5 hr sand 1.8 ml of ethyl benzoate was added to the mixture and kept with stirring at 90° C for 2 hr. Thereafter, a solid component was collected by filtration. Subsequently, the solid component was re-suspended in 200 ml of titanium tetrachloride again and heated at 90°C for 2 hr. Then, a solid component was collected by filtration. The solid component was thoroughly washed with purified hexane until a titanium compound liberated was not detected in a cleaning solution, and thereby a solid transition metal catalyst component (A) was prepared.

The solid transition metal catalyst component (A) contained 3.5 % by mass of titanium, 62.0 % by mass of chlorine, 17.0 % by mass of magnesium and 14.3 % by mass of ethyl benzoate in terms of atom. The solid transition metal catalyst component (A) was a granular catalyst having an average particle diameter of 1.0 µm and a particle size distribution that the geometrical standard deviation was 1.2.

### [Main Polymerization]

To a 2L internal volume autoclave, 1.0L of purified decane, 1.0 mmol of triisobutylaluminum and 0.05 mmol in terms of titanium atom of the solid transition metal catalyst component (A) were introduced. Thereafter, the temperature was elevated to 60° C and ethylene feeding was started. Ethylene was fed over 6 hr with keeping a total pressure of 2.5 kg/cm²G at 65°C. After completion of the polymerization, the temperature was decreased and the pressure was released and then a polymer was obtained in an amount of 130g (the amount of the resulting polymer was 1,896 g per 1 g of the solid transition metal catalyst component (A)).
The resulting polymer had an intrinsic viscosity [η] of 11. 6 dl/g. The polymer further had an average particle diameter of 9. µm, and a particle size distribution such that fine particles having a particle diameter of 0 to 40 µm were in an amount of 97.6% by mass. The resulting polymer contained 16 ppm of a titanium atom and 180 ppm of an aluminum atom.

### Example 2

To a 2L internal volume autoclave, 1.0L of purified decane, 1.0 mmol of triisobutylaluminum and 0.07 mmol in terms of titanium atom of the solid transition metal catalyst component (A) were introduced. Thereafter, the temperature was elevated to 60 °C and ethylene feeding was started. Ethylene was fed over 4 hr with keeping a total pressure of 2. 5 kg/cm² G at 65°C. After completion of the polymerization, the temperature was decreased and the pressure was released and then a polymer was obtained in an amount of 130g (the amount of the resulting polymer was 1,354 g per 1 g of the solid transition metal catalyst component (A)).
The resulting polymer had an intrinsic viscosity [η] of 12.0 dl/g. The polymer further had an average particle diameter of 8.0 µm, and a particle size distribution such that fine particles having a particle diameter of 0 to 40 µm were in an amount of 92.2 % by mass. The resulting polymer contained 36 ppm of a titanium atom and 180 ppm of an aluminum atom.

### Comparative Example 1

To a 2L internal volume autoclave, 1.0L of purified decane, 1.0 wool of triisobutylaluminum and 0.013 mmol in terms of titanium atom of the solid transition metal catalyst component (A) were introduced. Thereafter, the temperature was elevated to 60° C and ethylene feeding was started. Ethylene was fed over 4 hr with keeping a total pressure of 5.0 kg/cm² G at 65 °C. After completion of the polymerization, the temperature was decreased and the pressure was released and then a polymer was obtained in an amount of 130g (the amount of the resulting polymers was 7,292 g per 1 g of the solid transition metal catalyst component (A)).
The resulting polymer had an intrinsic viscosity [η] of 14.0 dl/g. The polymer had an average particle diameter of 14.7 µm, and a particle size distribution such that fine particles having a particle diameter of 0 to 40 µm were in an amount of 80.3 % by mass. The resulting polymer contained 6 ppm of a titanium atom and 180 ppm of an aluminum atom.

### Comparative Example 2

To a 2L internal volume autoclave, 1.0L of purified decane, 1.0 mmol of triisobutylaluminum and 0.006 mmol in terms of titanium atom of the solid transition metal catalyst component (A) were introduced. Thereafter, the temperature was elevated to 60°C and ethylene feeding was started. Ethylene was fed over 9 hr with keeping a total pressure of 6.0 kg/cm² G at 65°C. After completion of the polymerization, the temperature was decreased and the pressure was released and then a polymer was obtained in an amount of 130g (the amount of the resulting polymer was 15,799 g per 1 g of the solid transition metal catalyst component (A)).
The resulting polymer had an intrinsic viscosity [η] of 14.5 dl/g. The polymer further had an average particle diameter of 20.0 µm, and a particle size distribution such that fine particles having a particle diameter of 0 to 40 µm were in an amount of 73.6 % by mass. The resulting polymer contained 2 ppm of a titanium atom and 180 ppm of an aluminum atom.

### Example 3

The polymer prepared in Example 1 was put in a sealer jar and was heated at 170° C for 1 hr to prepare a uniform sintered filter free from molding unevenness.

### INDUSTRIAL APPLICABILITY

The ultra-high molecular weight polyolefin fine particles of the present invention are light in weight and have excellent abrasion resistance, impact resistance, chemical resistance and self-lubricating properties. Therefore, the ultra-high molecular weight polyolefin fine particles are favorably used for sintered filters, rubber modifiers, active carbon binders, mechanical parts, lining materials, sporting goods, cosmetics, carriers of medicines for tests such as medicines for pregnancy test and the like and carriers of microorganisms and cell culture.

The sintered filters obtainable by molding the ultra-high molecular weight polyolefin fine particles having a small particle diameter and a narrow particle size distribution according to the present invention have a uniform hole diameter size distribution and can be favorably used for various uses.

## Claims

1. Ultra-high molecular weight polyolefin fine particles comprising 1 to 50 ppm of a titanium atom and 1 to 1000 ppm of an aluminum atom, and having:
[A] an intrinsic viscosity [η], as measured in decalin at 135°C, of not less than 5 dl/g,
[B] an average particle diameter of not more than 20 µm, and
[C] a content of particles having a particle diameter of 0 to 40 µm of not less than 90 % by mass.

2. The ultra-high molecular weight polyolefin fine particles according to claim 1, which is obtainable by using a catalyst comprising:
(A) a solid transition metal catalyst component comprising a titanium atom and a magnesium atom, and
(B) an organoaluminum compound.

3. The ultra-high molecular weight polyolefin fine particles according to claim 1, which is obtainable by regulating the amount of a resulting polymer to not more than 7000 g per 1 g of the solid transition metal catalyst component (A) by using a catalyst comprising:
(A) a solid transition metal catalyst component comprising a titanium atom and a magnesium atom, and
(B) an organoaluminum compound.

4. The ultra-high molecular weight polyolefin fine particles according to any one of claims 1 to 3, which is obtainable by using a polymerization reactor equipped with a stirring blade and regulating a peripheral speed of a stirring blade tip to not less than 3.0 m/sec.

5. The ultra-high molecular weight polyolefin fine particles according to any one of claims 1 to 4, wherein the polyolefin is an ethylene polymer.

6. A process for producing ultra-high molecular weight polyolefin fine particles as claimed in claim 2, wherein the amount of a resulting polymer is regulated to be not more than 7000 g per 1 g of the solid transition metal catalyst component (A).

7. The process for producing ultra-high molecular weight polyolefin fine particles according to claim 6 wherein a polymerization reactor equipped with a stirring blade is used and the peripheral speed of a stirring blade tip is regulated to not less than 3.0 m/sec.

8. The process for producing ultra-high molecular weight polyolefin fine particles according to claim 6 or 7 wherein the polyolefin is an ethylene polymer.

9. A molded article comprising the ultra-high molecular weight polyolefin fine particles according to any one of claims 1 to 5.

10. A sintered filter obtainable by sintering molding the ultra-high molecular weight polyolefin fine particles according to any one of claims 1 to 5.

11. A modifier for rubber comprising the ultra-high molecular weight polyolefin fine particles according to any one of claims 1 to 5.

12. A binder for active carbon comprising the ultra-high molecular weight polyolefin fine particles according to any one of claims 1 to 5.

13. A cosmetic composition comprising the ultra-high molecular weight polyolefin fine particles according to any one of claims 1 to 5.
